# EUROPÄISCHE PATENTANMELDUNG

(11) **EP 3 875 957 A1**
(43) Veröffentlichungstag der Anmeldung: **08.09.2021**
(21) Anmeldenummer: 20178275.2
(22) Anmeldetag: 04.06.2020
(51) Int. Cl.: G01N 33/497

(54) **VERFAHREN UND VORRICHTUNG ZUR DIAGNOSE EINES VERDACHTS EINER VIRUSINFEKTION**

(30) Priorität: 04.03.2020 EP 20160935
(71) Anmelder: Elektrobit Automotive GmbH, 91058 Erlangen (DE)
(72) Erfinder: Strassenburg-Kleciak, Marek - c/o Continental Automotive GmbH, 65824 Schwalbach a. Ts. (DE)
(74) Vertreter: Continental Corporation

(57) **Zusammenfassung**

Die vorliegende Erfindung betrifft ein Verfahren und eine Vorrichtung zur Diagnose eines Verdachts einer Virusinfektion, insbesondere eines Fahrzeuginsassen. In einem ersten Schritt wird Ausatemluft des Fahrzeuginsassen gesammelt (S1). Die gesammelte Ausatemluft wird gefiltert (S2) und die gefilterte Ausatemluft kondensiert (S3). Das resultierende Kondensat kann zusätzlich konzentriert werden (S4). Anschließend wird das Kondensat für eine Untersuchung bereitgestellt (S5), in deren Rahmen das Kondensat auf Verdachtsanzeichen untersucht wird (S6). Abschließend werden die Untersuchungsergebnisse ausgewertet (S7).

## Beschreibung

Die vorliegende Erfindung betrifft ein Verfahren und eine Vorrichtung zur Diagnose eines Verdachts einer Virusinfektion, insbesondere eines Fahrzeuginsassen.

Das Risiko der Ansteckung mit einer leicht übertragbaren Viruserkrankung mit schweren gesundheitlichen Folgen zwingt Volkswirtschaften zu entschlossenen Maßnahmen, die die Bewegungsfreiheit einer Einzelperson oder von Personengruppen drastisch einschränken können. Das hat, wie man an der Pandemie mit SARS CoVid-2019, nach neuerer Bezeichnung SARS CoV 2, beobachten kann, gravierende wirtschaftliche Folgen. Die Technik der Temperaturmessung als Frühkontrollwerkzeug hat sich als wenig wirksam erwiesen, da zwischen der Infektion und dem Ausbruch der Erkrankung in der Regel zwei symptomfreie Wochen liegen. Die derzeit existierenden Tests, für die ein Abstrich erforderlich ist, bedürfen des Fachpersonals. Zudem sind sie in Hinblick auf die Eindämmung einer Epidemie nur bedingt wirksam, denn aus logistischen Gründen sind Massentests in Megametropolen nicht durchführbar.

Abhängig von der Art des Virus werden im Stand der Technik verschiedene Verfahren zum Nachweis des Virus verwendet. Dazu gehört die sogenannte kulturelle Virusanzucht, der Nachweis von Virusantigen, der Nukleinsäurenachweis (PCR: Polymerase Chain Reaction; Polymerase-Kettenreaktion) im Nasenabstrich oder Sputum, oder Bedside-Tests. Bei Coronaviren sind es Immunfluoreszenzverfahren, Neutralisationstests und ELISA Tests (ELISA: Enzyme-linked Immunosorbent Assay; enzymgekoppelter Immunadsorptionstest), wobei ELISA die größte Bedeutung hat. Der Nachweis akuter Coronainfektionen basiert fast immer auf sensitiven und spezifischen RT-PCR (RT-PCR: Real-Time Polymerase Chain Reaction; Echtzeit-Polymerase-Kettenreaktion) Techniken, meist unter Verwendung von Oligonukleotid-Primern, die in hochkonservierten Regionen des Replikase-Gens, vor allem der Polymerase- oder Helikase-Region binden.

Es ist eine Aufgabe der Erfindung, eine vereinfachte Diagnose eines Verdachts einer Virusinfektion zu ermöglichen, welche sich ohne großen Aufwand für die zu untersuchende Person anwenden lässt.

Diese Aufgabe wird durch ein Verfahren mit den Merkmalen des Anspruchs 1 und durch eine Vorrichtung mit den Merkmalen des Anspruchs 9 gelöst. Bevorzugte Ausgestaltungen der Erfindung sind Gegenstand der abhängigen Ansprüche.

Gemäß einem ersten Aspekt der Erfindung umfasst ein Verfahren zur Diagnose eines Verdachts einer Virusinfektion eines Fahrzeuginsassen die Schritte:
- Sammeln von Ausatemluft des Fahrzeuginsassen,
- Filtern der gesammelten Ausatemluft,
- Kondensieren der gefilterten Ausatemluft, und
- Untersuchen des Kondensats auf Verdachtsanzeichen.

Gemäß einem weiteren Aspekt der Erfindung weist eine Vorrichtung zur Diagnose eines Verdachts einer Virusinfektion eines Fahrzeuginsassen auf:
- eine Sammelvorrichtung zum Sammeln von Ausatemluft des Fahrzeuginsassen,
- zumindest eine Filterstufe zum Filtern der gesammelten Ausatemluft,
- eine Kondensationskammer zum Kondensieren der gefilterten Ausatemluft, und
- Mittel zum Untersuchen des Kondensats auf Verdachtsanzeichen.

Bei der erfindungsgemäßen Lösung wird die Ausatemluft eines Fahrzeuginsassen, insbesondere des Fahrers, aus einer geschlossener Autokabine für eine Diagnose gesammelt, gefiltert und kondensiert. Anschließend wird im Rahmen einer Analyse die Wahrscheinlichkeit einer Virusinfektion untersucht. Durch das Sammeln erhöht sich dabei die Anzahl der relevanten Partikel in der zu analysierenden Probe. Als Ergebnis der Analyse erfährt der Fahrzeuginsasse, ob er mit einer erhöhten Wahrscheinlichkeit ein Träger von Viren bestimmter Klassen ist oder nicht, sodass der Fahrzeuginsasse dementsprechend agieren kann. Die erfindungsgemäße Diagnosevorrichtung ist für ein Individuum zur Selbstkontrolle gedacht. Sie eignet sich aufgrund ihrer kompakten Ausführung gut für einen Einbau in ein Fahrzeug und zielt somit darauf, das Risiko der Ausbreitung einer Pandemie deutlich einzuschränken.

Gemäß einem Aspekt der Erfindung erfolgt das Filtern der gesammelten Ausatemluft in zwei Filterstufen. In der ersten Filterstufe kann die Ausatemluft des Fahrers von Staub gereinigt werden. In der zweiten Filterstufe können dann Partikel oberhalb einer gewünschten Größe gefiltert werden. Dies erlaubt es, die Anzahl unerwünschter Partikel für die nachfolgende Analyse zu reduzieren, d.h. es wird eine Verdichtung des Kondensats erzielt, sodass bessere Analyseergebnisse erzielt werden.

Gemäß einem Aspekt der Erfindung wird das Kondensat durch Entzug von Wasser konzentriert. Der Entzug von Wasser erfolgt dabei vorzugsweise mittels Umkehrosmose. Durch den Entzug von Wasser wird die Konzentration der nachzuweisenden Partikel in der Probe erhöht. Dies hilft dabei, diagnostische Lücken zu vermeiden. Eine diagnostische Lücke kommt zustande, wenn der nachzuweisende Analyt, z.B. Viruspartikel oder krankheitsspezifische Antikörper, nicht unmittelbar in ausreichender Konzentration vorliegt, um mit einem Erregernachweis oder dem Nachweis einer Immunantwort detektierbar zu sein.

Gemäß einem Aspekt der Erfindung umfasst das Untersuchen auf Verdachtsanzeichen zumindest eine mikroskopische Untersuchung des Kondensats. Die mikroskopische Untersuchung erfolgt dabei vorzugsweise mittels Lichtmikroskopie und/oder Röntgenmikroskopie. Typische Viren haben Abmessungen im Bereich von 15 nm bis knapp 1 µm. Die größeren Viren können somit noch mittels Lichtmikroskopie untersucht werden, die kleineren Viren mittels Röntgenstrahlen. Eine miniaturisierte Röntgenröhre, die für diesen Zweck einsetzbar ist, ist beispielsweise aus der DE 10 2015 001 440 A1 bekannt. Daneben kann grundsätzlich auch Elektronenmikroskopie eingesetzt werden, die prinzipiell für alle bekannten Virenabmessungen geeignet ist.

Gemäß einem Aspekt der Erfindung umfasst das Untersuchen auf Verdachtsanzeichen eine biochemische Untersuchung oder eine physikalische Untersuchung. Wie oben schon angeführt, ist erfindungsgemäß eine Verdichtung mit einem Filtersystem vorgesehen. Falls sich dennoch eine Vielzahl anderer, harmloser Viren in der gefilterten Fraktion befindet, ist es sinnvoll, alternative Nachweisverfahren zu verwenden, z.B. eine Western-Blot-Analyse oder einen Nukleinsäurenachweis mittels RT-PCR.

Gemäß einem Aspekt der Erfindung erfolgt eine Auswertung von Untersuchungsergebnissen mittels künstlicher Intelligenz. Die Untersuchungsergebnisse der verschiedenen Analysen werden bevorzugt jeweils einer KI-Auswertung unterzogen, d.h. die Auswertung basiert auf Algorithmen der künstlichen Intelligenz, insbesondere des maschinellen Lernens. Derartige Algorithmen eignen sich besonders gut für die Auswertung komplexer Datensätze.

Vorzugsweise wird eine erfindungsgemäße Vorrichtung in einem Fortbewegungsmittel eingesetzt, insbesondere in einem Kraftfahrzeug. Aufgrund ihrer kompakten Ausführung eignet sich die erfindungsgemäße Vorrichtung sehr gut für einen Einbau in einem Fahrzeug. Ein Vorteil ist dabei die präzise Erfassung des Gesundheitszustands des Fahrers. Durch die langfristige Messung, vorteilhafterweise zweimal am Tag, beispielsweise bei der Fahrt zur Arbeit und zurück, liegen bei den meisten Fahrern sehr gute Bedingungen für eine langfristige, validierbare Beobachtung von Veränderungen von dessen Allgemeinzustand vor. Neben der Diagnose auf eine mögliche Virusinfektion können auch weitere Krankheitsanzeichen überwacht werden, z.B. in Hinblick auf Diabetes, Asthma, etc. Diese ermöglicht es, dem Fahrer frühzeitig Hinweise auf eine angeratene ärztliche Kontrolle zu geben.

Weitere Merkmale der vorliegenden Erfindung werden aus der nachfolgenden Beschreibung und den angehängten Ansprüchen in Verbindung mit den Figuren ersichtlich.

### Figurenübersicht

- Fig. 1: zeigt schematisch ein Verfahren zur Diagnose eines Verdachts einer Virusinfektion eines Fahrzeuginsassen;
- Fig. 2: zeigt schematisch eine Vorrichtung zur Diagnose eines Verdachts einer Virusinfektion eines Fahrzeuginsassen;
- Fig. 3: zeigt schematisch einen Messaufbau für die Mikroskopie; und
- Fig. 4: illustriert schematisch eine Western-Blot-Analyse.

### Figurenbeschreibung

Zum besseren Verständnis der Prinzipien der vorliegenden Erfindung werden nachfolgend Ausführungsformen der Erfindung anhand der Figuren detaillierter erläutert. Gleiche Bezugszeichen werden in den Figuren für gleiche oder gleichwirkende Elemente verwendet und nicht notwendigerweise zu jeder Figur erneut beschrieben. Es versteht sich, dass sich die Erfindung nicht auf die dargestellten Ausführungsformen beschränkt und dass die beschriebenen Merkmale auch kombiniert oder modifiziert werden können, ohne den Schutzbereich der Erfindung zu verlassen, wie er in den angehängten Ansprüchen definiert ist.

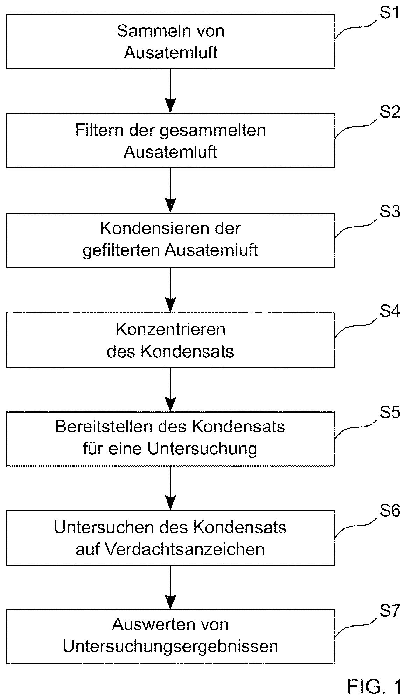

Fig. 1 zeigt schematisch ein Verfahren zur Diagnose eines Verdachts einer Virusinfektion eines Fahrzeuginsassen. In einem ersten Schritt wird Ausatemluft des Fahrzeuginsassen gesammelt S1. Die gesammelte Ausatemluft wird gefiltert S2 und die gefilterte Ausatemluft kondensiert S3. Das Filtern S2 der gesammelten Ausatemluft erfolgt dabei vorzugsweise in zwei Filterstufen. Das resultierende Kondensat kann zusätzlich konzentriert werden S4, beispielsweise durch Entzug von Wasser mittels Umkehrosmose. Anschließend wird das Kondensat für eine Untersuchung bereitgestellt S5, in deren Rahmen das Kondensat auf Verdachtsanzeichen untersucht wird S6. Dabei kann zumindest eine mikroskopische Untersuchung des Kondensats, z.B. mittels Lichtmikroskopie oder Röntgenmikroskopie, eine biochemische Untersuchung und/oder eine physikalische Untersuchung erfolgen. Abschließend werden die Untersuchungsergebnisse ausgewertet S7, vorzugsweise mittels Algorithmen der künstlichen Intelligenz.

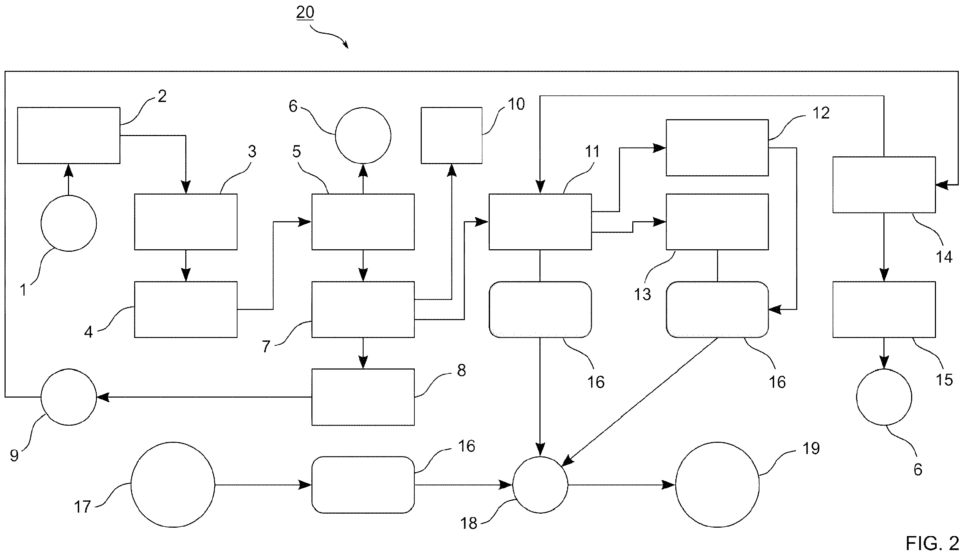

Fig. 2 zeigt schematisch eine Vorrichtung 20 zur Diagnose eines Verdachts einer Virusinfektion eines Fahrzeuginsassen. Die Atemluft 1 des Fahrers wird durch eine Sammelvorrichtung 2 gesammelt und durch eine erste Filterstufe 3, d.h. durch ein Vorfilter, von Staub gereinigt. In einer zweiten Filterstufe 4 werden Partikel oberhalb einer gewünschten Größe gefiltert. Die Größe der Filterporen ist dabei vorzugsweise an die nachzuweisenden Viren angepasst und kann z.B. 600 nm, im Fall von Coronaviren bevorzugt 250 nm betragen. Der Durchmesser der Viren des Typs SARS CoV 2 liegt bei etwa 60 nm bis 140 nm [1], sodass mit einer Porengröße von 250 nm eine gute Durchlässigkeit dieser Viren gewährleistet ist. Die filtrierte Atemluft 1 wird weiter durch eine Kondensationskammer 5 geführt. Die Abluft 6 wird abgeführt. Der kondensierten Flüssigkeit wird in einer Umkehrosmoseeinheit 7 mittels Umkehrosmosemembranen mithilfe einer Unterdruckpumpe 8 Wasser entzogen, das in einem Abwasserbehälter 9 gesammelt wird. Das resultierende Kondensat, d.h. der Teil der kondensierten Flüssigkeit, der nach dem Entzug des Wassers verbleibt, wird in eine Messkammer zur Analyse mittels eines Lichtmikroskops 11 weitergeleitet. Nach der Analyse mittels Lichtmikroskops 11 wird in derselben Kammer eine Analyse mittels eines Röntgenmikroskops 12 durchgeführt. Alternativ können an dieser Stelle auch andere Analysesysteme 13 zum Einsatz kommen, die weiter untern erläutert werden. Nach der Analyse entfernt ein Reinigungssystem 14 das Kondensat, wobei durch eine UVC-Lampe 15 eventuell vorhandene Viren abgetötet werden. Anschließend wird die Messkammer vom Reinigungssystem 14 mit dem osmotisch gereinigten Wasser aus dem Abwasserbehälter 9 gereinigt und der entsprechende Abfall vom Reinigungssystem 14 unter Nutzung der UVC-Lampe 15 in die Abluft 6 entfernt. Die Untersuchungsergebnisse der verschiedenen Analysen werden bevorzugt jeweils einer KI-Auswertung unterzogen, d.h. die zugeordneten Auswerteeinheiten 16 verwenden Ansätze der künstlichen Intelligenz. Dabei kann vorgesehen sein, dass eine der Auswerteeinheiten 16 weitere Vitalparameter 17 des Fahrzeuginsassen auswertet. Beispielsweise können Vitalparameter 17 wie die Körpertemperatur, Husten, Zittern, Schweiß, Änderungen der elektrischen Leitfähigkeit der Haut, eine Augenrötung, Verwirrung, eine verlangsamte Reaktionszeit oder Kurzatmigkeit, aber auch Änderungen in der Sprache, wie z.B. eine geänderte Tonlage oder geänderte Pausen zwischen den Worten bei einem Telefongespräch, Verdachtsmomente auf einen Infekt liefern. Im Rahmen einer Gesamtauswertung 18, die bevorzugt ebenfalls Ansätze der künstlichen Intelligenz nutzt, erfolgt schließlich eine Schätzung des Risikos einer Virusinfektion. Für diese Gesamtauswertung 18 können beispielsweise rekurrente neuronale Netzwerke (RNN: Recurrent Neural Network) genutzt werden, die bereits erfolgreich bei der Diagnose von Erkrankungen auf Grundlage verschiedenster Vitalparameter eingesetzt wurden [2]. Das Ergebnis 19 der Gesamtauswertung 18 wird an den Fahrzeuginsassen ausgegeben.

Die Vorrichtung 20 befindet sich erfindungsgemäß vorzugsweise in einer thermischen Ummantelung mit einem Temperaturregler. Viren sind des Öfteren anfällig für Temperaturschwankungen, weshalb es sinnvoll ist, die in der Fachliteratur beschriebene optimale Temperatur einzuhalten.

Das Messgerät kann vorteilhaft als eine separate Einheit außerhalb vom Fahrzeug ausgeführt werden. Dazu kann das mittels der Umkehrosmoseeinheit 7 verdichtete Kondensat in einem Kondensatbehältnis 10 für die Untersuchungen bereitgestellt werden.

Die Analyse durch ein Röntgenmikroskop 12 kann vorteilhaft durch andere Analysetechniken ersetzt oder ergänzt werden. Beispiele dafür sind biochemische Verfahren, in denen Substanzen eingesetzt werden, die mit Viren einer bestimmten Art reagieren, oder physikalische Verfahren. Beispielsweise kommen das aus [3] bekannte Pamono-Verfahren, ein Western-Blot-Verfahren oder ein Echtzeit-PCR-Verfahren infrage. Echtzeit-PCR-Verfahren sind eine sehr sensitive Methode zur Detektion von humanen Coronaviren. Beispielsweise können in diesem Zusammenhang universelle Oligonukleotide herangezogen werden, die die Amplifikation konservierter Genombereiche erlauben, z. B. Regionen der ORFs 1a/1b. Durch ein entsprechendes PCR-Primer-Design kann durch Amplifikation typspezifischer, variabler Bereiche eine sehr hohe Präzision der Diagnose erreicht werden.

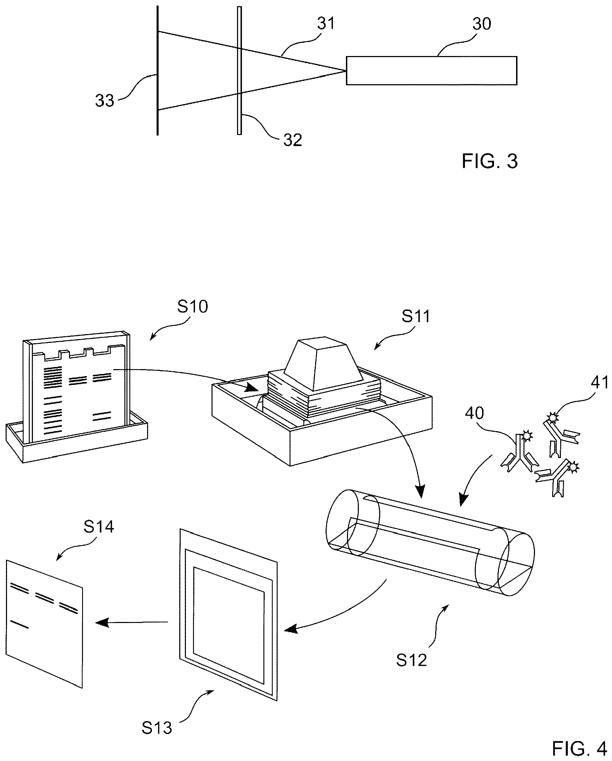

Fig. 3 zeigt schematisch einen Messaufbau für die Mikroskopie, der genutzt werden kann, wenn die kondensierte Ausatemluft des Fahrers mittels bei Röntgenmikroskopie untersucht wird. Dabei bezeichnet 30 eine Strahlungsquelle, die Röntgenstrahlung 31 emittiert. Die emittierte Röntgenstrahlung 31 durchleuchtet eine Probe 32, d.h. ein Volumen, in dem sich die kondensierte, vorgefilterte Ausatemluft befindet. Ein Szintillator 33 wandelt die Röntgenstrahlen 31 in sichtbares Licht um.

Fig. 4 illustriert schematisch eine Western-Blot-Analyse. Die Western-Blot-Analyse, auch als Immunoblot bekannt, wird verwendet, um ein bestimmtes Protein in einer Zelle, einem Gewebe, einem Organ oder einer Körperflüssigkeit nachzuweisen. Die Technik basiert auf der Reaktion eines Antikörpers mit einem Protein, das auf einer dünnen Membran immobilisiert ist. Zunächst erfolgt eine Separierung S10 der Proteine. Dazu wird eine Probe mit einem Detergens solubilisiert und die Proteine werden dann durch Elektrophorese in einem Polyacrylamidgel getrennt. Nach der Elektrophorese wird das Gel für einen Transferschritt S11 neben eine dünne synthetische Membran gelegt, die eine starke Affinität zu Proteinen aufweist. In der Figur sind das Gel und die Membran zwischen absorbierenden Papierblättern in einem Löschtank angeordnet. Diese Anordnung ermöglicht es dem Puffer, über das Gel und durch die dünne Membran zu fließen. Infolgedessen werden die Proteine im Gel durch Kapillarwirkung auf die Membran übertragen. Die Übertragung der Proteine auf die Membran kann auch durch elektrischen Strom erfolgen.

Nach dem Transferschritt S11 wird die Membran mit einem Antikörper 40 gegen ein bestimmtes Protein inkubiert S12. Dieser Antikörper kann in einem Versuchstier wie einer Maus oder einem Kaninchen oder in Zellen als monoklonaler Antikörper hergestellt werden. Der Antikörper 40 kann an ein Enzym 41 gekoppelt sein, das dann zum Nachweis des Antikörpers 40 auf der Membran verwendet werden kann. In dem gezeigten Beispiel ist der Antikörper 40 an Meerrettichperoxidase gekoppelt. Die Membran wird mit einem Substrat inkubiert S12, das nach Reaktion mit diesem Enzym in eine Lumineszenzverbindung umgewandelt wird. Anschließend wird neben der Membran eine Röntgenfolie angeordnet und eine Autoradiographie S13 durchgeführt. Nach der Entwicklung und Fixierung des Autoradiogramms können schließlich die einzelnen Proteine sichtbar gemacht werden S14. In einer Variation der Technik wird ein unmarkierter erster Antikörper verwendet, um das Protein auf der Membran zu binden, und ein zweiter Antikörper, der gegen den ersten Antikörper gerichtet ist, wird zum Nachweis verwendet. Der Hauptvorteil der Western-Blot-Analyse besteht darin, dass keine Isotopenmarkierung von Proteinen erforderlich ist und sie mit Geweben und Organen sowie kultivierten Zellen verwendet werden kann.

Wird die Western-Blot-Analyse verwendet, ist eine Kammer mit einer Anzahl von mehreren Tests von Vorteil, wobei für jedes Testverfahren eine neue Western-Blot-Einheit verwendet werden kann. Beispielsweise kann eine austauschbare Kartusche mit 24 Tests verwendet werden, die automatisch in eine Messkammer verschoben und nach den Messungen entfernt werden können. Die Kartusche mit Tests kann in der Ausführung analog zu einem typischen Mechanismus eines Gewehrmagazins gebaut werden.

Aus [4] ist bekannt, dass die Isolierung von humanen Coronaviren und ihre Kultivierung direkt aus Patientenmaterial oft sehr schwierig ist. HCoV-229E kann in humanen diploiden Zelllinien, wie MRC-5, vermehrt werden, während HCoV-OC43 meist Organkultursysteme benötigt. Laboradaptierte OC43-Stämme replizieren allerdings in verschiedenen Zelllinien. SARS-CoV und HCoV-NL63 wurden u.a. in Nierenzellen von Meerkatzen erfolgreich kultiviert, während HCoV-HKU1 bislang noch nicht in Zellkultur vermehrt werden konnte. Obwohl die Elektronenmikroskopie einen wichtigen Beitrag bei der Identifizierung und Charakterisierung von Coronaviren geleistet hat, dient sie nicht standardmäßig zu diagnostischen Zwecken, da andere Partikel in klinischen Proben ähnlich wie Coronaviren aussehen können und darüber hinaus ein hoher Titer im zu untersuchenden Material benötigt wird. Bei einer Vielzahl diagnostischer Verfahren werden Nukleinsäuretechniken routinemäßig zum schnellen und sicheren Erregernachweis herangezogen. So stellen RT-PCR Assays eine sehr sensitive Methode zur HCoV-Detektion dar. Weniger als fünf RNA-Kopien pro Reaktionsansatz können reproduzierbar nachgewiesen werden. Allerdings muss das Probenmaterial von guter Qualität sein und entsprechend sorgfältig gehandhabt werden. In diesem Zusammenhang kommt ein wichtiger Aspekt der erfindungsgemäßen Lösung zum Tragen, indem längeres Sammeln und Filtrieren der Partikel zu einem besseren Ergebnis führt.

### Referenzen

[1] M. Cascella et al.: "Features, Evaluation and Treatment Coronavirus (COVID-19)" https://www.ncbi.nlm.nih.gov/books/NBK554776/
[2] Z. Lipton et al.: "Learning to Diagnose with LSTM Recurrent Neural Networks" https://arxiv.org/pdf/1511.03677v6.pdf
[3] F. Weichert et al.: "GPGPU-basierte Echtzeitdetektion von Nanoobjekten mittels Plasmonen-unterstützter Mikroskopie" http://ceur-ws.org/Vol-715/bvm2011_10.pdf
[4] Christian Lehmann: "Humane Coronaviren: Entwicklung und Anwendung eines Immunoassays und Untersuchungen zur zellvermittelten Immunität", Dissertation, Regensburg 2008 https://epub.uni-regensburg.de/12135/1/Dissertation_Lehmann.pdf

## Patentansprüche

1. Verfahren zur Diagnose eines Verdachts einer Virusinfektion eines Fahrzeuginsassen, mit den Schritten:
- Sammeln (S1) von Ausatemluft (1) des Fahrzeuginsassen,
- Filtern (S2) der gesammelten Ausatemluft (1),
- Kondensieren (S3) der gefilterten Ausatemluft (1), und
- Untersuchen (S6) des Kondensats auf Verdachtsanzeichen.

2. Verfahren gemäß Anspruch 1, wobei das Filtern (S2) der gesammelten Ausatemluft (1) in zwei Filterstufen (3, 4) erfolgt.

3. Verfahren gemäß Anspruch 1 oder 2, wobei das Kondensat durch Entzug (S4) von Wasser konzentriert wird.

4. Verfahren gemäß Anspruch 3, wobei der Entzug (S4) von Wasser mittels Umkehrosmose erfolgt.

5. Verfahren gemäß einem der vorherigen Ansprüche, wobei das Untersuchen (S6) auf Verdachtsanzeichen zumindest eine mikroskopische Untersuchung des Kondensats umfasst.

6. Verfahren gemäß Anspruch 5, wobei die zumindest eine mikroskopische Untersuchung mittels Lichtmikroskopie oder Röntgenmikroskopie erfolgt.

7. Verfahren gemäß einem der vorherigen Ansprüche, wobei das Untersuchen (S6) auf Verdachtsanzeichen eine biochemische Untersuchung oder eine physikalische Untersuchung umfasst.

8. Verfahren gemäß einem der vorherigen Ansprüche, wobei eine Auswertung (S7) von Untersuchungsergebnissen mittels künstlicher Intelligenz erfolgt.

9. Vorrichtung (20) zur Diagnose eines Verdachts einer Virusinfektion eines Fahrzeuginsassen, mit:
- einer Sammelvorrichtung (2) zum Sammeln (S1) von Ausatemluft (1) des Fahrzeuginsassen,
- zumindest einer Filterstufe (3, 4) zum Filtern (S2) der gesammelten Ausatemluft (1),
- einer Kondensationskammer (5) zum Kondensieren (S3) der gefilterten Ausatemluft (1), und
- Mitteln (11, 12, 13) zum Untersuchen (S6) des Kondensats auf Verdachtsanzeichen.

10. Vorrichtung (20) gemäß Anspruch 9, wobei die Vorrichtung (20) eine Testkammer mit einer Vielzahl von Testeinheiten aufweist.
